# EUROPÄISCHE PATENTSCHRIFT

(11) **EP 0 849 596 B1**
(45) Veröffentlichungstag und Bekanntmachung des Hinweises auf die Patenterteilung: **15.02.2006**
(21) Anmeldenummer: 97120361.7
(22) Anmeldetag: 20.11.1997
(51) Int. Cl.: G01N 33/543, G01N 33/569

(54) **Immundissoziation zur Verbesserung der immunchemischen Bestimmung eines Analyten**
Immunodissociation for improving the immunochemical determination of an analyte
Immunodissociation pour amélioration de la détermination immunochimique d'un analyte

(30) Priorität: 19.12.1996 DE 19653074; 03.01.1997 DE 19700134
(43) Veröffentlichungstag der Anmeldung: 24.06.1998
(73) Patentinhaber: Dade Behring Marburg GmbH, 35001 Marburg (DE)
(72) Erfinder: Brust, Stefan, Dr., 35041 Marburg (DE)

(56) Entgegenhaltungen:
- EP-A- 0 572 845
- HASHIDA ET AL.: "Detection of antibody IgG to HIV-1 in urine by ultrasensitive enzyme immunoassay ...." JOURNAL OF CLINICAL LABORATORY ANALYSIS, Bd. 8, Nr. 2, 1994, Seiten 86-95, XP002092767 new york, ny

## Beschreibung

Die vorliegende Erfindung betrifft ein Verfahren zur immunchemischen Bestimmung eines oder mehrerer Analyten in einer Probe mittels eines immobilisierten spezifischen, mit dem Analyten bioaffin wechselwirkenden Rezeptors R1 und eines ebenfalls mit dem Analyten bioaffin wechselwirkenden spezifischen Rezeptors R2, welcher in der Regel markiert ist. Das erfindungsgemäße Verfahren ist dadurch gekennzeichnet, daß ein Rezeptor R3, der eine oder mehr als eine spezifische Bindungsstelle zum Analyten aufweist, zugegeben wird und die entstehenden Immunkomplexe ganz oder teilweise dissoziiert, danach reassoziiert und anschließend detektiert werden. Gemäß einer weiteren Ausführungsform wird zusätzlich zum Rezeptor R3 ein Rezeptor R4 eingesetzt, der eine Affinität gegenüber R3 aufweist und an der Festphase immobilisiert ist, wobei die entstehenden Immunkomplexe ganz oder teilweise dissoziiert, danach reassoziiert und anschließend detektiert werden.

Übliche immunologische Verfahren zur Diagnose von Erkrankungen, die mit der Bildung von spezifischen Antikörper gegen einen Krankheitsauslöser, wie Viren, Bakterien, Allergene, Autoantigene oder bestimmte Arzneimittel einhergehen, beruhen auf der Fähigkeit dieser Antikörper zur Komplexbildung mit antigenen Strukturen des Auslösers.

Bei bestimmten Ausführungsformen dieser Verfahren, die allgemein als heterogene Immunoassays bezeichnet werden, wird eine auf den Gehalt an beispielsweise spezifischen Antikörpern (Analytantikörpern) zu prüfende Probe mit antigenen Strukturen der Krankheitsauslöser in Kontakt gebracht, wobei diese antigenen Strukturen an geeigneten, bekannten Trägermaterialien immobilisiert sind. In der Probe enthaltene Analytantikörper werden als Immunkomplex an die an den Trägermaterialien immobilisierten antigenen Strukturen des Krankheitsauslösers gebunden und nachgewiesen. Zum Nachweis können Nachweisantikörper bzw. andere spezifische Rezeptoren (z.B. Protein A), die zur Komplexbindung mit dem Analytantikörper der Probe befähigt sind, verwendet werden.

Das Nachweisreagenz trägt in der Regel eine Markierung, welche die Menge des gebundenen Antikörpers meßtechnisch erfaßbar macht.

Gebräuchliche Markierungen sind radioaktive Isotope, Enzyme, fluoreszierende, phosphoreszierende oder lumineszierende Substanzen, Substanzen mit stabilen ungepaarten Elektronen, Latexpartikel, Magnetpartikel, Metallsole und Erythrozyten.

Bei diesen Verfahren sind sowohl einstufige wie auch mehrstufige Nachweismethoden bekannt. Jeder Verfahrensschritt wird üblicherweise mit einem Trennprozess (Waschschritt) beendet.

Die sehr einfach durchzuführende Technik der Einschritt-Methode bei heterogenen Immunoassays ist allerdings nicht zum Nachweis aller Krankheitsmarker geeignet. Oft müssen aus technischen Gründen zwei- oder mehrstufige Verfahren angewandt werden.

Bekannt sind auch mehrstufige Verfahren, die als Immunkomplex-Transfer-Enzymimmunoassays bezeichnet werden (S. Hashida et al., *Journal of Clinical Laboratory Analysis* **8**:86-95 (1994)). Dabei wird der gesamte aus Festphasenantigen, spezifischem Antikörper und markiertem Konjugatantigen bestehende Immunkomplex von der Festphase abgelöst. Nach Überpipettieren auf eine antikörperbindende Festphase wird der gesamte Immunkomplex fixiert und detektiert.

Diese Methoden sind sehr spezifisch, haben allerdings den Nachteil, daß die nachzuweisenden Krankheitsauslöser oder gegen sie gerichtete Antikörper, welche im ersten Schritt mit dem immobiliserten, spezifischen Rezeptor einen Komplex eingegangen sind, in den darauffolgenden Reaktionsschritten in einer dem Fachmann bekannten Rückreaktion sich zum Teil wieder aus dem Komplex lösen können und sich somit der Nachweisreaktion entziehen, wodurch beispielsweise die Sensitivität eingeschränkt wird.

Die diagnostische Leistungsfähigkeit solcher mehrstufiger Verfahren wird insbesondere dann besonders stark eingeschränkt, wenn die Rückreaktionsrate zwischen immobilisiertem Rezeptor und dem nachzuweisenden Agens hoch ist. Dies ist beispielsweise bei niederaffinen

Antikörpern gegen Krankheitsauslöser oder Arzneimittel der Fall. Dem Fachmann bekannt sind diese Effekte besonders bei Verfahren zum Nachweis von häufig mutierenden Krankheitsauslösem oder Krankheitsmarkern, welche mit dem immobiliserten spezifischen Rezeptor nach Mutation eine geringe Wechselwirkung zeigen.

Bereits in der EP 0 572 845 wurde offenbart, daß die Rückreaktionsrate durch Zusatz eines weiteren Rezeptors gegen Strukturmerkmale des nachzuweisenden Agens erheblich reduziert wird. Dieser Rezeptor muß mehr als eine bindungsfähige Stelle für das nachzuweisenden Agens aufweisen und darf den immunchemischen Nachweis des Agens nicht stören.

Trotz deutlich reduzierter Rückreaktion ermöglicht es auch diese Methode nicht, spezielle niederaffine Antikörper gegen Krankheitsauslöser oder Arzneimittel sicher und mit hoher Sensitivität nachzuweisen.

Daher bestand die Aufgabe, Reagenzien zu finden, die diese Nachteile nicht aufweisen.

Überraschenderweise wurde festgestellt, daß bedingt durch einen Immundissoziations- und nachfolgenden Reassoziationsschritt des bereits gebildeten Immunkomplexes aus immobilisiertem spezifischen Rezeptor R1 und damit bioaffin wechselwirkendem Analyten A und dem gegen diesen Analyten gerichteten spezifischen Rezeptor R2, welcher in der Regel markiert ist, eine Signalsteigerung zu verzeichnen ist, wenn zusätzlich ein Rezeptor R3 zugegeben wird. Dieser reduziert den Anteil an nicht festphasenfixiertem Analyten dadurch, daß er, ohne die Immunreaktion zwischen Analyt A und Rezeptor R1 oder Analyt A und Rezeptor R2 zu unterdrücken, mehr als einen Analyten zu binden vermag.

Gemäß einer weiteren Ausführungsform wird zusätzlich zu R3 ein weiterer immobilisierter Rezeptors R4 zugegeben, welcher mit R3 bioaffin wechselwirken kann. R4 reduziert durch Fixierung des Immunkomplexes, bestehend aus dem Analyten A und dem Rezeptor R3, an die Festphase den Anteil an nicht immobiliserten Immunkomplexen. Hierbei muß der spezifische Rezeptor R3 allerdings nicht, wie in der obigen Ausführungsform, mehr als eine spezifische Bindungsstelle zum Analyten A aufweisen. Durch diese zusätzliche Fixierung des Analyten A und somit auch des Rezeptors R2, der in der Regel die Markierung trägt, wird die Signalausbeute deutlich gesteigert und letztlich die Sensitivität des Assays für den Analyten A erhöht.

Gemäß einer weiteren Ausführungsform wird der Rezeptor R4 auf einer zweiten Festphase immobilisiert und werden die dissoziierten Immunkomplexe, beispielsweise R3-A-R2, von der ersten Festphase auf die zweite Festphase übertragen, wo der Reassoziationschritt und auch die Detektion von R2 erfolgen.

Das nachzuweisende Analyt A im Sinne dieser Erfindung kann sowohl ein beispielsweise durch einen Krankheitsauslöser induzierter Antikörper, als auch ein Antigen, wie beispielsweise der Krankheitsauslöser selbst sein.

Gegenstand der Erfindung ist daher ein Verfahren zur immunologischen Bestimmung eines oder mehrerer Analyten mittels:
eines spezifischen Rezeptors R1, wobei dieser spezifische Rezeptor R1 an einem Träger immobilisert ist und das Ausmaß der Bindung des Analyten A an den spezifische Rezeptor R1 mittels eines weiteren spezifische Rezeptor R2, der direkt oder indirekt eine Markierung trägt, bestimmt wird, wobei in dem Verfahren ein Dissoziationsschritt durchgeführt wird, dadurch gekennzeichnet, daß ein Rezeptor R3, der eine oder mehr als eine bindungsfähige Stelle für das nachzuweisende Agens A aufweist, zugesetzt wird, der keine Affinität zu dem immobiliserten spezifische Rezeptor R1 aufweist und nicht markiert ist und das Ausmaß der Bindung des Analyten A an den spezifischen Rezeptor R2, der direkt oder indirekt eine Markierung trägt, nach Reassoziation bestimmt wird.

Weiterer Gegenstand der Erfindung ist ein Verfahren zur immunologischen Bestimmung eines oder mehrerer Analyten mittels:
eines spezifischen Rezeptors R1, wobei dieser spezifische Rezeptor R1 an einem Träger immobilisert ist und das Ausmaß der Bindung des Analyten A an den spezifischen Rezeptor R1 mittels eines weiteren spezifischen Rezeptors R2, der direkt oder indirekt eine Markierung trägt, bestimmt wird, wobei in dem Verfahren ein Dissoziationsschritt durchgeführt wird, dadurch gekennzeichnet, daß zusätzlich zum Bindungsfaktor R3, der hierbei nicht mehr als eine einzige bindungsfähige Stelle für das nachzuweisende Agens aufweisen muß, ein weiterer spezifischer immobilisierter oder immobilisierbarer Rezeptor R4 zugesetzt wird, der eine Affinität zu R3 aufweist und das Ausmaß der Bindung des Analyten A an den spezifischen Rezeptor R2, der direkt oder indirekt eine Markierung trägt, nach Reassoziation bestimmt wird.

Ein weiterer Gegenstand der Erfindung ist ein Verfahren zur immunologischen Bestimmung eines oder mehrerer Analyten mittels:
eines spezifischen Rezeptors R1, wobei dieser spezifische Rezeptor R1 an einem Träger immobilisert ist und das Ausmaß der Bindung des Analyten A an den spezifischen Rezeptor R1 mittels eines weiteren spezifischen Rezeptors R2, der direkt oder indirekt eine Markierung trägt, bestimmt wird, wobei in dem Verfahren ein Dissoziationsschritt durchgeführt wird, dadurch gekennzeichnet, daß zusätzlich zum Bindungsfaktor R3, der hierbei nicht mehr als eine einzige bindungsfähige Stelle für das nachzuweisende Agens aufweisen muß, die disoziierenten Immunkomplexe, beispielsweise R3-A-R2, auf eine zweite Festphase übertragen werden, die einen weiteren spezifischen immobilisierten oder immobilisierbaren Rezeptor R4 enthält, der eine Affinität zu R3 aufweist und das Ausmaß der Bindung des Analyten A an den spezifischen Rezeptor R2, der direkt oder indirekt eine Markierung trägt, nach Reassoziation bestimmt wird.

Nicht markiert bedeutet im Sinne der vorliegenden Erfindung, daß R3 entweder kein Label oder zumindestens nicht dasjenige Label trägt, mit dem das Ausmaß der Bindung des Analyten an den spezifischen Rezeptor bestimmt wird.

Die Verfahren, in denen die immobilisierten spezifischen Rezeptoren R4 verwendet werden können, sind in allen ihren Ausführungsformen dem Fachmann bekannt. Wichtig ist, daß die erfindungsgemäßen Verfahren in geeigneter Form bei allen immunchemischen Verfahren, bei denen in einem ersten Schritt eine immunchemische oder vergleichbare Bindung eines Analyten an einen bevorzugterweise immobilisierten, spezifischen Rezeptor erfolgt und in einem zweiten, aber nicht unbedingt zeitlich getrennten Schritt, ein direkter oder indirekter Nachweis erfolgt, eingesetzt werden können.

Bevorzugt werden diese Methoden in dem als Sandwich-ELISA dem Fachmann bekannten Verfahren eingesetzt, wobei bevorzugterweise als Markierungssystem ein Enzym, bevorzugterweise mit einem chromogenen oder fluorogenen Substrat oder ein lumineszentes Label verwendet wird. Die gewählte Ausführungsform beeinflußt jedoch primär die Verwendungsmöglichkeiten der erfindungsgemäßen Verfahren nicht.

Als Festphasen werden bevorzugterweise Mikrotitrationsplatten, Magnetpartikel, Latexpartikel oder matrixchemische Testelemente, wie z.B. Fasern oder Membranen enthaltende Testmodule verwendet.

Dem Fachmann ist bekannt, daß immunchemische Verfahren, wie oben beschrieben, zur gleichzeitigen Bestimmung von unterschiedlichen Analyten, beispielsweise HIV 1 (HIV = Humanes Immundefizienzvirus), HIV 2, einzeln oder miteinander kombiniert, oder HIV 1 und/oder HIV 2, kombiniert mit HCV (Hepatitis C-Virus oder Hepatitis C-Virusantigen), eingesetzt werden können. Analyten in dem vorstehenden Sinne können sowohl die viralen Antigene, als auch die dagegen gerichteten Antikörper sein. Solche Ausführungsformen sind hiermit auch eingeschlossen.

Dissoziation in Sinne der Erfindung beinhaltet alle chemischen und physikalischen Methoden, die es ermöglichen, bioaffine Wechselwirkungen reversibel zu lösen oder herabzusetzen. Dem Fachmann sind chemische Substanzen bekannt, die bei der immunchemischen Reinigung von Proteinen und Antikörpern zum Einsatz kommen wie beispielsweise Rhodanid, Harnstoff oder Guanidin. Auch durch immunologische Verfahren, wie beispielsweise Kompetition mit geeigneten immunologisch aktiven Substanzen, kann eine Dissoziation herbeigeführt werden. Weiterhin kann durch Einsatz saurer oder basischer Lösungen eine Dissoziation erzwungen werden. Physikalische Methoden der Dissoziation sind dem Fachmann ebenfalls bekannt: beispielsweise Temperaturunterschiede, Mikrowellen oder Ultraschall.

Reassoziation im Sinne der vorliegenden Erfindung bedeutet u.a. die Herbeiführung nicht dissoziierender Bedingungen, beispielsweise durch Neutralisieren, Verdünnen oder einfach durch Rückgängigmachen der vorher herrschenden dissoziierenden Bedingungen, wie Beseitigen der weiter oben erwähnten Temperaturunterschiede oder durch Abschalten des Mikrowellen- oder Ultraschallgenerators.

Rezeptoren R3 im Sinne der Erfindung sind spezifische Bindungspartner, die eine oder mehr als eine bioaffine Bindungsstelle zum Analyten A aufweisen. Weiterhin können sie eine Wechselwirkung mit dem Rezeptor R4 eingehen. Bindungspartner oder Komponenten dieses Rezeptors R3 können Konjugate von Antikörpern oder Antikörper selbst sein. Diese Antikörper oder Antikörperfragmente bzw. Konjugate daraus können gegen A oder aber auch gegen A und R4 gerichtet sein. Der Rezeptor R3 kann auch aus einem Antikörper oder Antikörperfragment bestehen, welcher/welches mit einem Strukturelement gekoppelt ist, gegen das der Rezeptor R4 gerichtet ist.

Rezeptoren R4 im Sinne der Erfindung sind spezifische Bindungspartner, die eine oder mehrere bioaffine Bindungsstellen zu R3 aufweisen. Rezeptoren oder Komponente dieses Rezeptors können Konjugate von Antikörpern/Antikörperfragmenten oder Antikörper/-fragmente selbst sein. Diese Antikörper/-fragmente bzw. Konjugate daraus können gegen R3 gerichtet sein oder aber antigene Strukturen aufweisen, die von Bindungsstellen des Rezeptors R3 erkannt werden. R4 kann auch aus einem Protein bestehen, welches mit einem Strukturelement gekoppelt ist, gegen das R3 gerichtet ist.

Außerdem ist Gegenstand der Erfindung ein Reagenz zur Verwendung im oben genannten Verfahren.

Bevorzugte Kombinationen von Rezeptoren zum spezifischen Nachweis von Antigenen (Analyt) sind:
R1: Antikörper, der gegen den Analyten gerichtet ist
R2: Mit einem Label versehener Antikörper, der gegen den Analyten gerichtet ist
R3: Antikörper, der gegen den Analyten A gerichtet ist.
Bevorzugte Kombinationen von Rezeptoren zum spezifischen Nachweis von Antigenen (Analyt) sind:
R1: Antikörper, der gegen den Analyten A gerichtet ist
R2: Mit einem Label versehener Antikörper, der gegen den Analyten A gerichtet ist
R3: Antikörper, der gegen den Analyten A gerichtet ist
R4: Antikörper, der gegen den Antikörper R3 gerichtet ist.

Im Falle des Antigennachweises wird bevorzugterweise ein Antikörper bzw. ein Antikörperfragment als Rezeptor R3 verwendet, der nicht dasselbe Epitop erkennt wie der Festphasenantikörper R1 oder der Konjugatantikörper R2.

Bevorzugte Kombinationen von Rezeptoren zum Nachweis spezifischer Antikörper (Analyt) sind:
R1: Antigen, gegen das der Analyt A gerichtet ist
R2: Mit einem Label versehenes Antigen, gegen das der Analyt A gerichtet ist
R3: Antikörper, der gegen den Analyten A gerichtet.

Bevorzugte Kombinationen von Rezeptoren zum Nachweis spezifischer Antikörper (Analyt) sind:
R1: Antigen, gegen das der Analyt A gerichtet ist
R2: Mit einem Label versehenes Antigen, gegen das der Analyt A gerichtet ist
R3: Antikörper, der gegen den Analyt A gerichtet ist
R4: Antikörper, der gegen den Antikörper R3 gerichtet ist.

Besonders bevorzugte Kombinationen von Rezeptoren zum Nachweis spezifischer Antikörper (Analyt) sind:
R1: Antigen, gegen das der Analyt A gerichtet ist
R2: Mit einem Label versehenes Antigen, gegen das der Analyt A gerichtet ist
R3: Antikörper, der gegen den Analyten A gerichtet ist und zusätzliche nicht antikörpereigene Epitope trägt
R4: Antikörper, der gegen ein zusätzliches nicht antikörpereigenes Epitop von Antikörper R3 gerichtet ist.

Dem Fachmann sind Methoden zur Herstellung solcher Konjugate, die jeweils aus einem Antikörper und einem zusätzlichen nicht antikörpereigenen Antigen (z.B. Biotin oder Digoxigenin) bestehen, bekannt. Solche Konjugate können, unter Erhalt der bioaffinen Funktion ihrer Ausgangsmaterialien, beispielsweise durch Verknüpfung mittels chemischer Reagenzien oder durch bioaffine Wechselwirkung hergestellt werden. Es können auch Hybridmoleküle durch chemische Synthese, die Hybridoma-Technik oder gentechnologische Methoden erzeugt werden.

Das erfindungsgemäße Reagenz kann Verwendung finden in einer Vielzahl von Verfahren der Human- und Veterinärdiagnostik. Als Beispiele sollen angeführt werden zwei- oder mehrstufige Teste zu Nachweis von Antikörpern verschiedener Immunglobulinklassen gegen Strukturmerkmale von Viren (z.B. Viren der Hepatitis A, B und C, sowie verschiedener HIV-Typen), von bakteriellen und parasitären Erregern sowie von allergischen Erkrankungen. Weitere Beispiele sind der Nachweis von Krankheitserregern wie Viren (z.B. Hepatitis B-Virus), Bakterien, Parasiten und Allergenen, wie auch von Markern von Krankheiten (z.B. Tumormarkern) in mehrstufigen Nachweisverfahren.

Die vorliegende Erfindung wird außerdem durch die nachfolgenden Beispiele, welche jedoch keine Einschränkung der allgemeineren Lehre bedeuten sollen sowie durch die Patentansprüche erläutert.

### Beispiel 1

### 1a) Herstellung der Festphase

Mikrotitrationsplatten Typ B (Fa. Nunc, Roskilde, Dänemark) werden mit 100 µl je Vertiefung Beschichtungslösung (600 µg/l rekombinantes gp41 [Behringwerke AG, Marburg, BRD] und 10 mg/ml monoklonaler Antikörper gegen Biotin [Behringwerke AG, Marburg, BRD] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1 % Tween 20, pH 7.2) gewaschen. Die über Kieselgel getrockneten Mikrotitrationsplatten sind unter Luftausschluß etwa 1 Jahr stabil.

### 1b) Herstellung des Konjugates

10 mg HIV 1-gp41-Peptid (IAF Biochem, Laval, Kanada) werden in 1 ml Eisessig/Wasser (50:50, v/v) gelöst. Nach Neutralisieren mit 5N Natronlauge wird mit einem 10fach molaren Überschuß an N-γ-Maleimidobutyrylsuccinimid versetzt und 1 Stunde bei Raumtemperatur inkubiert. Das nicht umgesetzte heterobifunktionelle Reagenz wird durch Gelfiltration (Sephadex G-25) mit 100 mM Natriumphosphat, 5 mM Nitriloessigsäure, pH 6.0 abgetrennt.

10 mg Meerrettich-Peroxidase (Boehringer Mannheim, Mannheim, BRD) werden in 10 ml 10 mM Natriumphosphat, 100 mM Kochsalz, pH 8.0 mit 100fach molarem Überschuß an 2-Iminothiolan 1 Stunde bei Raumtemperatur inkubiert. Anschließend wird freies Modifierungsreagenz durch Gelfiltration (Sephadex G-25) mit 100 mM Natriumphosphat, 5 mM Nitrilotriessigsäure, pH 6.0 entfernt.

Beide Eluate (SH-aktivierte Peroxidase und Maleimid-modifizierte HIV 1-Peptid) werden vereinigt und über Nacht bei Raumtemperatur inkubiert.

Nach Abstoppen mit 1/10 Vol 100 mM N-Ethyl-Maleimid wird das Konjugat durch Gelfiltration (Sephadex G-25) von nicht abreagiertem HIV 1-Peptid gereinigt. Nach Ankonzentrieren (2 mg/ml) wird das Konjugat bei -20°C gelagert.

### 1c) Herstellung des biotinylierten Antikörpers

10 mg Monoklonaler Antikörper gegen humanes Immunglobulin G [Behringwerke AG, Marburg, BRD] in 10 ml 10 mM Natriumphosphat, 100 mM Kochsalz, pH 8.0 werden mit 10fach molarem Überschuß an N-Hydroxysuccinimid-X-Biotin in 10 mM Natriumphosphat, 100 mM Kochsalz, pH 8.0 für 1 Stunde bei Raumtemperatur inkubiert. Anschließend wird freies Modifierungsreagenz durch Gelfiltration (Sephadex G-25) mit 100 mM Tris, 5 mM Nitrilotriessigsäure, pH 7.0 entfernt.

### Beispiel 2: Anwendung des erfindungsgemäßen Reagenzes

### 2a) Enzymimmunoassay zum Nachweis von HIV 1-Antikörpern (Referenzsystem I)

Ein Enzymimmunassay zum Nachweis von Anti-HIV 1 wird wie folgt durchgeführt:

In die Vertiefungen der nach Beispiel 1a hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 125 µl des nach Beispiel 1b hergestellten Konjugates (1:1000 in 0.1 M Tris/HCl, 1 mM Glycin, 0.2 % Albumin, 0.4 % Pluronic F64, 1 mg/l monoklonaler Antikörper gegen humanes. Immunglobulin G, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

### 2b) Enzymimmunoassay zum Nachweis von HIV 1-Antikörpern (Erfindungsgemäßes System I)

Ein Enzymimmunassäy zum Nachweis von Anti-HIV 1 wird wie folgt durchgeführt:
In die Vertiefungen der nach Beispiel 1a hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 100 µl des Dissoziationspuffers (5 mM Glycin/HCl, pH 2,5) für 30 min bei RT zugegeben. Anschließend werden ohne zu Waschen 25 µl nach Beispiel 1b hergestellten Konjugates (1:200 in 0.5 M Tris/HCl, 1% Albumin, 2% Pluronic F64, 5 mg/l monoklonaler Antikörper gegen humanes Immunglobulin G, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

Anti-HIV 1 positive sowie Anti-HIV 1 positive Proben mit ungewöhnlich niedriger Reaktivität und anti-HIV negative Seren wurden sowohl im Referenzsystem wie auch im erfindungsgemäßen Enzymimmunoassay untersucht.

Die Resultate (Extinktionseinheiten) der Untersuchung finden sich in der Tabelle 1.

**Tabelle 1:**

| **Proben-Bezeichnung** | **Anti-HIV Status** | **Verdünnung** | **Bemerkun g** | **Referenz-system I** | **Erfindungsgemäßes System I** |
|---|---|---|---|---|---|
| **Negative Kontrolle** | Negativ | nativ | | 0,098 | 0,103 |
| **Positive Kontrolle** | Positiv | 1:4000 | hochaffin | 1,575 | 1,412 |
| **9111/39** | Positiv | 1:100 | niederaffin | 1,211 | 1,653 |
| **9111/39** | Positiv | 1:200 | niederaffin | 0,520 | 0,886 |
| **9111/46** | Positiv | 1:50 | niederaffin | 0,528 | 0,927 |
| **9111/46** | Positiv | 1:100 | niederaffin | 0,310 | 0,489 |
| **9205/19** | Positiv | 1:8000 | hochaffin | 0,906 | 0,877 |
| **BS 1-5** | Negativ | nativ | | 0,076 | 0,089 |
| **BS 1-17** | Negativ | nativ | | 0,077 | 0,078 |
| **BS 1-33** | Negativ | nativ | | 0,090 | 0,075 |
| **BS 1-59** | Negativ | nativ | | 0,083 | 0,091 |

Deutliche Unterschiede in der Signalbildung der beiden Testsystemen ist insbesondere bei niederaffinen Proben (z.B. 9111/46) zu erkennen. Die Sensitivität bezüglich dieser Proben wird fast um das Doppelte gegenüber dem Referenzsystem I gesteigert. Proben mit hoher Affinität zum Beschichtungsantigen, welche auch im Referenzsystem I bereits in hoher Verdünnung erkannt werden, erfahren im erfindungsgemäßen System I keinen Signalzuwachs. Anti-HIV negative Seren reagieren in beiden Testsystemen vergleichbar.

### Beispiel 3: Anwendung des erfindungsgemäßen Reagenzes

### 3a) Enzymimmunoassay zum Nachweis von HIV 1-Antikörpern (Referenzsystem II)

Ein Enzymimmunassay zum Nachweis von Anti-HIV 1 wird wie folgt durchgeführt:

In die Vertiefungen der nach Beispiel 1a hergestellten Mikrotitrationsplatten werden 25 µl Probenpuffer (0.3 M Tris/HCl, 1 % Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 125 µl des nach Beispiel 1b Konjugates (1:1000 in 0.1 M Tris/HCl, 1 mM Glycin, 0.2 % Albumin, 0.4 % Pluronic F64, 1 mg/l nach Beispiel 1c) biotinylierter monoklonaler Antikörper gegen humanes Immunglobulin G, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

### 3b) Enzymimmunoassay zum Nachweis von HIV 1-Antikörpern (Erfindungsgemäßes System II)

Ein Enzymimmunassay zum Nachweis von Anti-HIV 1 wird wie folgt durchgeführt:

In die Vertiefungen der nach Beispiel 1 a hergestellten Mikrotitrationsplatten werden 25 µl Probenpuffer (0.3 M Tris/HCl, 1 % Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 100 µl des Dissoziationspuffers (5 mM Glycin/HCl, pH 2,5) für 30 min bei RT zugegeben. Anschließend werden ohne zu Waschen 25 µl nach Beispiel 1b hergestellten Konjugates (1:200 in 0.5 M Tris/HCl, 1 % Albumin, 2% Pluronic F64, 5 mg/l nach Beispiel 1c) biotinylierter monoklonaler Antikörper gegen humanes Immunglobulin G, pH 8.1) einpipettiert. Mit 4 weiteren Waschschritten wird die 30minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

Anti-HIV 1 positive sowie Anti-HIV 1 positive Proben mit ungewöhnlich niedriger Reaktivität und anti-HIV negative Seren wurden sowohl im Referenzsystem wie auch im erfindungsgemäßen Enzymimmunoassay untersucht.

Die Resultate (Extinktionseinheiten) der Untersuchung finden sich in der Tabelle 2.

**Tabelle 2:**

| **Probenbezeichnung** | **Anti-HIV Status** | **Verdünnung** | **Bemerkun g** | **Referenzsystem II** | **Erfindungsgemäßes System II** |
|---|---|---|---|---|---|
| **Negative Kontrolle** | Negativ | nativ | | 0,149 | 0,140 |
| **Positive Kontrolle** | Positiv | 1:800 | hochaffin | 0,997 | 1,834 |
| **9111/39** | Positiv | 1:100 | niederaffin | 1,813 | >2,500 |
| **9111/39** | Positiv | 1:200 | niederaffin | 0,988 | 1,714 |
| **9111/46** | Positiv | 1:50 | niederaffin | 0,694 | 0,800 |
| **9111/46** | Positiv | 1:100 | niederaffin | 0,341 | 0,502 |
| **9205/19** | Positiv | 1:8000 | hochaffin | 0,997 | 1,834 |
| **BS 1-5** | Negativ | nativ | | 0,098 | 0,090 |
| **BS 1-17** | Negativ | nativ | | 0,086 | 0,086 |
| **BS 1-33** | Negativ | nativ | | 0,089 | 0,080 |
| **BS 1-59** | Negativ | nativ | | 0,084 | 0,079 |

Deutliche Unterschiede in der Signalbildung der beiden Testsystemen ist insbesondere bei niederaffinen Proben (z.B. 9111/39) zu erkennen. Die Sensitivität bezüglich dieser Proben wird um das Doppelte gegenüber dem Referenzsystem II gesteigert. Proben mit hoher Affinität zum Beschichtungsantigen, welche bereits im Referenzsystem II in hoher Verdünnung erkannt werden, erfahren im erfindungsgemäßen System II einen weiteren Signalzuwachs. Anti-HIV negative Seren reagieren in beiden Testsystemen vergleichbar.

## Patentansprüche

1. Verfahren zur Bestimmung eines Analyten, worin
(1) eine den zu bestimmenden Analyten gegebenenfalls enthaltende Probe mit einem an einer festen Phase immobilisierten Rezeptor R1, welcher eine Bindungsaffinität zum Analyten A besitzt, in Kontakt gebracht wird, so daß ein Immunkomplex R1-A entsteht;
(2) ein oder mehrere Waschschritte durchgeführt werden;
(3) ein markierter Rezeptor R2, welcher mindestens eine Bindungsstelle mit Bindungsaffinität zum Analyten A besitzt und ein nicht markierter Rezeptor R3, welcher mehr als eine Bindungsstelle mit Bindungsaffinität zum Analyten A besitzt, zugesetzt werden, wobei die Zugabe der Rezeptoren R2 und R3 in beliebiger Reihenfolge erfolgen kann und wobei zunächst dissoziierende Bedingungen, die zur Spaltung von Immunkomplexen führen und anschließend nicht dissoziierende Bedingungen, welche die Reassoziation und Neubildung von Immunkomplexen erlauben, herbeigeführt werden, so daß immobilisierte Immunkomplexe enthaltend A, R1, R2 und R3 entstehen;
(4) die Menge der an der festen Phase immobilisierten Markierung, welche ein Maß für die Menge des an der festen Phase immobilisierten Analyten ist, in geeigneter Weise detektiert wird.

2. Verfahren gemäß Anspruch 1, worin (1) an der festen Phase zusätzlich ein Rezeptor R4 immobilisiert ist, welcher mindestens eine Bindungsstelle mit Bindungsaffinität zum Rezeptor R3 besitzt, worin (2) der Rezeptor R3 mindestens eine Bindungsstelle mit Affinität zum Analyten A besitzt und worin (3) Immunkomplexe enthaltend einerseits A, R2 und R3 und andererseits R1 und/ oder R4 entstehen.

3. Verfahren zur Bestimmung eines Analyten, worin
(1) eine den zu bestimmenden Analyten gegebenenfalls enthaltende Probe mit einem an einer ersten festen Phase immobilisierten Rezeptor R1, welcher eine Bindungsaffinität zum Analyten A besitzt, in Kontakt gebracht wird, so daß ein Immunkomplex R1-A entsteht;
(2) ein oder mehrere Waschschritte durchgeführt werden;
(3) ein Dissoziationsschritt durchgeführt wird, der zur Spaltung des Immunkomplexes R1-A führt;
(4) ein markierter Rezeptor R2, welcher mindestens eine Bindungsstelle mit Bindungsaffinität zum Analyten A besitzt und ein nicht markierter Rezeptor R3, welcher mindestens eine Bindungsstelle mit Bindungsaffinität zum Analyten A besitzt, zugesetzt werden, wobei die Zugabe der Rezeptoren R2 und R3 in beliebiger Reihenfolge erfolgen kann und wobei nicht dissoziierende Bedingungen, welche die Neubildung von Immunkomplexen erlauben, herbeigeführt werden und wobei der Analyt A und die Rezeptoren R2 und R3 mit einer zweiten festen Phase in Kontakt gebracht, an welcher ein Rezeptor R4 immobilisiert ist, welcher mindestens eine Bindungsstelle mit Bindungsaffinität zum Rezeptor R3 besitzt, so daß immobilisierte Immunkomptexe enthaltend A, R2, R3 und R4 entstehen und
(5) die Menge der an der festen Phase immobilisierten Markierung, welche ein Maß für die Menge des an der festen Phase immobilisierten Analyten ist, in geeigneter Weise detektiert wird.

4. Verfahren gemäß Anspruch 1, worin R1 und R3 jeweils gegen den Analyten gerichtete Antikörper oder Antikörperfragmente sind und R2 ein markierter, gegen den Analyten gerichteter Antikörper oder ein markiertes, gegen den Analyten gerichtetes Antikörperfragment ist.

5. Verfahren gemäß Anspruch 2 oder 3, worin R1 und R3 jeweils gegen den Analyten gerichtete Antikörper oder Antikörperfragmente sind, R2 ein markierter, gegen den. Analyten gerichteter Antikörper oder markiertes, gegen den Analyten gerichtetes Antikörperfragment und R4 ein gegen R3 gerichteter Antikörper oder gegen R3 gerichtetes Antikörperfragment ist.

6. Verfahren gemäß Anspruch 5, worin R3 ein anderes Epitop erkennt als R1 oder R2.

7. Verfahren gemäß Anspruch 1, worin der Analyt ein Antikörper, R1 ein Antigen, gegen das der Analyt gerichtet ist, R2 ein markiertes Antigen, gegen das der Analyt gerichtet ist und R3 ein gegen den Analyten gerichteter Antikörper oder gegen den Analyten gerichtetes Antikörperfragment ist.

8. Verfahren gemäß Anspruch 2 oder 3, worin der Analyt ein Antikörper, R1 ein Antigen, gegen das der Analyt gerichtet ist, R2 ein markiertes Antigen, gegen das der Analyt gerichtet ist, R3 ein gegen den Analyten gerichteter Antikörper oder gegen den Analyten gerichtetes Antikörperfragment und R4 ein gegen R3 gerichteter Antikörper oder gegen R3 gerichtetes Antikörperfragment ist.

9. Verfahren gemäß Anspruch 8, worin R3 ein zusätzliches nicht antikörpereigenes Epitop trägt und R4 gegen das zusätzliche nicht antikörpereigene Epitop von R3 gerichtet ist.

10. Verfahren gemäß einem oder mehreren der vorherigen Ansprüche zur Bestimmung von Antikörpern gegen HIV.

11. Diagnostikum zur Durchführung des Verfahrens gemäß Anspruch 3 oder Anspruch 4, enthaltend einen an einer festen Phase immobilisierten Rezeptor R1 mit Bindungsaffinität zu einem Analyten A, einen markierten Rezeptor R2 mit mindestens einer Bindungsstelle mit Bindungsaffinität für A, einen nicht markierten Rezeptor R3 mit mindestens einer Bindungsstelle mit Bindungsaffinität für A und einen an einer festen Phase immobilisierten. Rezeptor R4 mit mindestens einer Bindungsstelle mit Bindungsaffinität für R3.

## Claims

1. A process for determining an analyte, which comprises
(1) bringing a sample, which may contain the analyte to be determined, into contact with a receptor R1, which is immobilized on a solid phase and which possesses a binding affinity for the analyte A, such that an immune complex R1-A is produced;
(2) carrying out one or more washing steps;
(3) adding a labeled receptor R2, which possesses at least one binding site having binding affinity for the analyte A, and an unlabeled receptor R3, which possesses more than one binding site having binding affinity for the analyte A, with it being possible for the.receptors R2 and R3 to be added in any order, and with initially dissociating conditions, which lead to the cleavage of immune complexes, and then non-dissociating conditions, which permit reassociation and neoformation of immune complexes, being brought about such that immobilized immune complexes containing A, R1, R2 and R3 are produced;
(4) detecting, in a suitable manner, the quantity of the label which is immobilized on the solid phase, which quantity is a measure of the quantity of the analyte which is immobilized on the solid phase.

2. The process as claimed in claim 1, wherein (1) a receptor R4 is additionally immobilized on the solid phase, which receptor R4 possesses at least one binding site having binding affinity for the receptor R3, wherein (2) the receptor R3 possesses at least one binding site having affinity for the analyte A, and wherein (3) immune complexes which contain A, R2 and R3, on the one hand, and R1 and/or R4, on the other, are produced.

3. A process for determining an analyte, which comprises
(1) bringing a sample, which may contain the analyte to be determined, into contact with a receptor R1, which is immobilized on a solid phase and which possesses a binding affinity for the analyte A, such that an immune complex R1-A is produced;
(2) carrying out one or more washing steps;
(3) carrying out a dissociation step, which leads to the cleavage of the immune complex R1-A;
(4) adding a labeled receptor R2, which possesses at least one binding site having binding affinity for the analyte A, and an unlabeled receptor R3, which possesses at least one binding site having binding affinity for the analyte A, with it being possible for the receptors R2 and R3 to be added in any order and with non-dissociating conditions, which permit the neoformation of immune complexes, being brought about and with the analyte A and the receptors R2 and R3 being brought into contact with a second solid phase, on which a receptor R4 is immobilized, which receptor R4 possesses at least one binding site having binding affinity for the receptor R3, such that immobilized immune complexes containing A, R2, R3 and R4 are produced; and
(5) detecting, in a suitable manner, the quantity of the label which is immobilized on the solid phase, which quantity is a measure of the quantity of the analyte which is immobilized on the solid phase.

4. The process as claimed in claim 1, wherein R1 and R3 are in each case antibodies, or antibody fragments, which are directed against the analyte and R2 is a labeled antibody which is directed against the analyte or a labeled antibody fragment which is directed against the analyte.

5. The process as claimed in claim 2 or 3, wherein R1 and R3 are in each case antibodies, or antibody fragments, which are directed against the analyte, R2 is a labeled antibody which is directed against the analyte or a labeled antibody fragment which is directed against the analyte and R4 is an antibody which is directed against R3 or an antibody fragment which is directed against R3.

6. The process as claimed in claim 5, wherein R3 recognizes an epitope which is different from that recognized by R1 or R2.

7. The process as claimed in claim 1, wherein the analyte is an antibody, R1 is an antigen against which the analyte is directed, R2 is a labeled antigen against which the analyte is directed and R3 is an antibody which is directed against the analyte or an antibody fragment which is directed against the analyte.

8. The process as claimed in claim 2 or 3, wherein the analyte is an antibody, R1 is an antigen against which the analyte is directed, R2 is a labeled antigen against which the analyte is directed, R3 is an antibody which is directed against the analyte or an antibody fragment which is directed against the analyte and R4 is an antibody which is directed against R3 or an antibody fragment which is directed against R3.

9. The process as claimed in claim 8, wherein R3 carries an additional epitope which is not antibody-intrinsic and R4 is directed against the additional epitope of R3, which epitope is not antibody-intrinsic.

10. The process as claimed in one or more of the preceding claims for determining antibodies against HIV.

11. A diagnostic agent for carrying out the process as claimed in claim 3 or claim 4, containing a receptor R1 which is immobilized on a solid phase and has binding affinity for an analyte A, a labeled receptor R2 which has at least one binding site having binding affinity for A, an unlabeled receptor R3 which has at least one binding site having binding affinity for A, and a receptor R4 which is immobilized on a solid phase and has at least one binding site having binding affinity for R3.

## Revendications

1. Procédé pour la détermination d'un analyte, dans lequel
(1) on met un échantillon, contenant éventuellement l'analyte à déterminer, en contact avec un récepteur R1 immobilisé sur une phase solide, qui a une affinité de liaison pour l'analyte A, ce par quoi on obtient un complexe immun R1-A ;
(2) on effectue une ou plusieurs étapes de lavage ;
(3) on ajoute un récepteur R2 marqué qui comporte au moins un site de liaison à affinité de liaison pour l'analyte A, et un récepteur R3 non marqué qui comporte plus d'un site de liaison à affinité de liaison pour l'analyte A, l'addition des récepteurs R2 et R3 pouvant s'effectuer en un ordre quelconque et en provoquant d'abord des conditions de dissociation qui conduisent à la dissociation des complexes immuns et ensuite des conditions de non-dissociation qui permettent la ré-association et la néoformation de complexes immuns, ce par quoi on obtient des complexes immuns immobilisés contenant A, R1, R2 et R3 ;
(4) on détecte de façon appropriée la quantité du marqueur immobilisé sur la phase solide, qui est une mesure de la quantité de l'analyte immobilisé sur la phase solide.

2. Procédé selon la revendication 1, dans lequel (1) on immobilise sur la phase solide un récepteur R4 qui comporte au moins un site de liaison à affinité de liaison pour le récepteur R3, dans lequel (2) le récepteur R3 comporte au moins un site de liaison à affinité pour l'analyte A et dans lequel (3) il se forme des complexes immuns contenant d'une part A, R2 et R3 et d'autre part R1 et/ou R4.

3. Procédé pour la détermination d'un analyte, dans lequel
(1) on met un échantillon, contenant éventuellement l'analyte à déterminer, en contact avec un récepteur R1 immobilisé sur une première phase solide, qui a une affinité de liaison pour l'analyte A, ce par quoi on obtient un complexe immun R1-A ;
(2) on effectue une ou plusieurs étapes de lavage ;
(3) on effectue une étape de dissociation qui conduit à la dissociation du complexe immun R1-A ;
(4) on ajoute un récepteur R2 marqué qui comporte au moins un site de liaison à affinité de liaison pour l'analyte A, et un récepteur R3 non marqué qui comporte au moins un site de liaison à affinité de liaison pour l'analyte A, l'addition des récepteurs R2 et R3 pouvant s'effectuer en un ordre quelconque et en provoquant des conditions de non-dissociation qui permettent la néoformation de complexes immuns, et on met l'analyte A et les récepteurs R2 et R3 en contact avec une deuxième phase solide sur laquelle est immobilisé un récepteur R4 qui comporte au moins un site de liaison à affinité de liaison pour le récepteur R3, ce par quoi on obtient des complexes immuns immobilisés contenant A, R2, R3 et R4 ; et
(4) on détecte de façon appropriée la quantité du marqueur immobilisé sur la phase solide, qui est une mesure de la quantité de l'analyte immobilisé sur la phase solide.

4. Procédé selon la revendication 1, dans lequel R1 et R3 sont des anticorps ou des fragments d'anticorps dirigés chacun contre l'analyte et R2 est un anticorps marqué dirigé contre l'analyte ou un fragment d'anticorps marqué dirigé contre l'analyte.

5. Procédé selon la revendication 2 ou 3, dans lequel R1 et R3 sont des anticorps ou des fragments d'anticorps dirigés chacun contre l'analyte, R2 est un anticorps marqué dirigé contre l'analyte ou un fragment d'anticorps marqué dirigé contre l'analyte et R4 est un anticorps dirigé contre R3 ou un fragment d'anticorps dirigé contre R3.

6. Procédé selon la revendication 5, dans lequel R3 reconnaît un autre épitope que R1 ou R2.

7. Procédé selon la revendication 1, dans lequel l'analyte est un anticorps, R1 est un antigène contre lequel est dirigé l'analyte, R2 est un antigène marqué contre lequel est dirigé l'analyte et R3 est un anticorps dirigé contre l'analyte ou un fragment d'anticorps dirigé contre l'analyte.

8. Procédé selon la revendication 2 ou 3, dans lequel l'analyte est un anticorps, R1 est un antigène contre lequel est dirigé l'analyte, R2 est un antigène marqué contre lequel est dirigé l'analyte, R3 est un anticorps dirigé contre l'analyte ou un fragment d'anticorps dirigé contre l'analyte et R4 est un anticorps dirigé contre R3 ou un fragment d'anticorps dirigé contre R3.

9. Procédé selon la revendication 8, dans lequel R3 porte un épitope supplémentaire n'appartenant pas à l'anticorps et R4 est dirigé contre l'épitope supplémentaire de R3 n'appartenant pas à l'anticorps.

10. Procédé selon une ou plusieurs des revendications précédentes, pour la détermination d'anticorps contre le VIH.

11. Agent de diagnostic pour la mise en oeuvre du procédé selon une ou plusieurs des revendications 1 à 10.
